(19) Europäisches Patentamt
European Patent Office
Office européen des brevets

(11) Publication number: **0 147 537**
**A1**

# (12) EUROPEAN PATENT APPLICATION

(21) Application number: 84111272.5

(22) Date of filing: 21.09.84

(51) Int. Cl.[4]: C 07 C 149/437
C 07 C 147/14, C 07 C 147/12
A 61 K 31/185, A 61 K 31/27

(30) Priority: 23.09.83 US 535019
23.09.83 US 535445

(43) Date of publication of application:
10.07.85 Bulletin 85/28

(84) Designated Contracting States:
IT

(71) Applicant: SCHERING CORPORATION
2000 Galloping Hill Road
Kenilworth, New Jersey 07033(US)

(72) Inventor: Vashi, Dhiru B.
20-05, Deer Creek Road
Plainsboro New Jersey(US)

(72) Inventor: Clark, Jeffrey N.
Rural Road No. 1 Ivins Drive
New Egypt NewJersey(US)

(72) Inventor: Lindo, Neil A.
209, Commonwealth Avenue
New Providence New Jersey(US)

(74) Representative: von Kreisler, Alek, Dipl.-Chem. et al,
Deichmannhaus am Hauptbahnhof
D-5000 Köln 1(DE)

(54) **Phenylguanidines.**

(57) This invention relates to phenyl guanidine compounds. Also disclosed are methods for preparing the compounds, compositions containing them, and methods for their use as anthelmintics.

EP 0 147 537 A1

PHENYLGUANIDINES

This invention relates to novel phenylguanidines, to methods for preparing and using them, and to compositions containing them. Compounds of the invention have anthelmintic activity.

The compounds of the present invention are represented by the formula

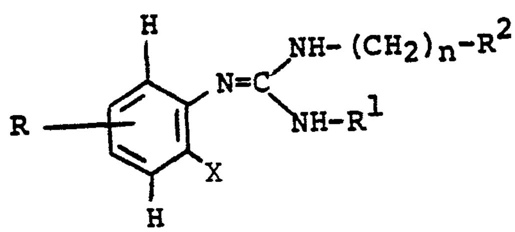

I

including their tautomers at the guanidine group, and the pharmaceutically acceptable salts thereof, wherein

X is a group $NO_2$ or $NR^8R^9$,

R is hydrogen, $-OR^3$, $-S(O)_mR^3$, $-\overset{\overset{O}{\|}}{C}-\overset{\overset{R^3}{|}}{N}-R^4$, $-\overset{\overset{O}{\|}}{C}-R^3$, halogen or $-CF_3$;

$R^1$ is hydrogen, $-\overset{\overset{O}{\|}}{C}-\overset{\overset{R^5}{|}}{N}-R^6$, or $-\overset{\overset{O}{\|}}{C}-OR^7$;

$R^2$ is $-SO_3H$, $-OSO_3H$, -COOH, $-PO_3H_2$, or $-OPO_3H_2$;

$R^3$ and $R^4$ are independently hydrogen, loweralkyl, loweralkoxyloweralkyl, hydroxyloweralkyl, cycloloweralkyl, phenyl or substituted phenyl, benzyl or substituted benzyl (wherein there are 1, 2 or 3 substituents on the substituted phenyl or substituted benzyl selected independently from halogen, loweralkyl, loweralkoxy, haloloweralkyl, or loweralkoxyloweralkyl), or 5 or 6 membered heterocycles having 1 or 2 heteroatoms selected independently from oxygen, nitrogen and sulfur;

$R^5$, $R^6$ and $R^7$ are independently hydrogen, loweralkyl, loweralkoxyloweralkyl, hydroxyloweralkyl, phenyl or substituted phenyl, and benzyl or substituted benzyl (where there are 1, 2 or 3 substitutents on the substituted phenyl or substituted benzyl selected independently from halogen, loweralkyl, loweralkoxy, haloloweralkyl, or loweralkoxyloweralkyl) except that $R^7$ cannot be hydrogen;

$R^8$ and $R^9$ are independently hydrogen loweralkyl, loweralkylcarbonyl, loweralkoxycarbonyl, loweralkoxyloweralkyl, phenyl or substituted phenyl, benzoyl or substituted benzoyl, phenoxycarbonyl or substituted phenoxycarbonyl (wherein there are 1, 2 or 3 substituents on the substituted phenyl, substituted benzoyl or substituted phenoxycarbonyl independently selected from halogen, lower alkyl, loweralkoxy, trifluoromethyl, and haloloweralkyl), carboxy-substituted lower alkyl, or sulfo-substituted lower alkyl;

$\underline{n}$ is 1 to 6; and

$\underline{m}$ is 0, 1 or 2;

provided that, when R is loweralkoxy, loweralkylthio, loweralkylsulfoxy, loweralkylsulfonyl, phenoxy, phenylsulfonyl, phenylthio or phenylsulfoxy,

$R^1$ is loweralkoxycarbonyl, loweralkoxyloweralkoxycarbonyl or hydroxyloweralkoxycarbonyl, and

X is nitro, loweralkylcarbonylamino, phenylacetamido, benzamido, or benzamido substituted with loweralkyl or halogen,
then $R^2$ is $PO_3H_2$ or $OPO_3H_2$.

This proviso preferably applies not only for the foregoing values of R and X but also when R is $OR^5$ (where $R^5$ is as defined above) and when X (independently of R) is $NH_2$.

As used herein, "lower alkyl" means straight or branched alkyl chains of 1 to 6 carbon atoms, e.g. methyl, ethyl, n-propyl, n-butyl, iso-butyl, and hexyl. Similarly, "lower alkoxy" means alkoxy groups having 1 to 6 carbon atoms, e.g. methoxy, ethoxy, propoxy, iso-butoxy, and pentoxy. "Cycloloweralkyl" means alkyl rings of 3 to 6 members, i.e. cyclopropyl, cyclobutyl, cyclopentyl, and cyclohexyl. "Halogen" means fluorine, chlorine, bromine and iodine. "Haloloweralkyl" means loweralkyl groups substituted by 1 to 3 halogen atoms, e.g. trifluoromethyl, dichloromethyl and chloroethyl.

Examples of heterocycles defined in $R^3$ and $R^4$ are pyridine, furan, thiophene, pyrimidine, piperazine and thiazole. All positional isomers are contemplated, e.g., 2-, 3-, and 4-pyridine, 2- and 3-furan.

The pharmaceutically acceptable salts contemplated include metal salts, e.g. alkali and alkali earth metal salts such as sodium, potassium and calcium, and other physiologically acceptable salts, e.g. trisamine, alkyl ammonium salts such as N-methylglucamine, ethanolamine, diethanolamine, triethanolamine, pyridinium and procaine, and tetraalkylammonium salts such as those produced with tetramethylammonium or tetraethylammonium ions.

Also included within the scope of the invention are the tautomers at the guanidine group,

i.e. 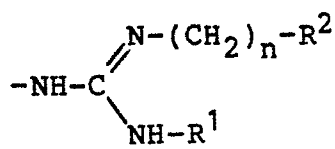 and 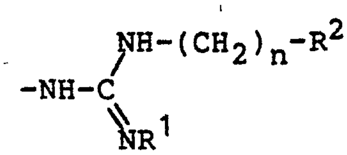

Preferred compounds include those wherein R is a group $CF_3$ or $R^3SO_m$, where *m* and $R^3$ are as defined above; $R^3$ is preferably a lower alkyl group, expecially an n-propyl group, and *m* is preferably O. The group R ($CF_3$ or $R^3SO_m$) is preferably at the 5-position. X is preferably $NO_2$ or $NH_2$; $R^2$ is preferably $SO_3H$; *n* is preferably 2; and $R^1$ is preferably hydrogen or $CONR^5R^6$, especially wherein $R^5$ and $R^6$ are both lower alkyl, e.g. methyl groups. Representative preferred compounds include

N-[(2-nitro-5-propylsulfonyl)phenyl]-N'-(2-ethyl sulfonic acid)guanidine;

N-[(2-nitro-5-propylthio)phenyl]-N'-(2-ethyl sulfonic acid)guanidine,

N-[(2-nitro-5-propylsulfinyl)phenyl]-N'-(2-ethyl sulfonic acid)guanidine,

N-methoxycarbonyl-N'-[(2-dimethylamino-5-propylthio)phenyl]-N"-(2-ethyl sulfonic acid)guanidine,

N-methoxycarbonyl-N'-[(2-dimethylamino-5-propylthio)phenyl]-N"-(3-propyl sulfonic acid)guanidine,

N-methoxycarbonyl-N'-[(2-(N"'-methyl-N"'-(2-ethyl sulfonic acid)amino)-5-propylthio)phenyl]-N"-(2-ethyl sulfonic acid)guanidine, and

N-methoxycarbonyl-N'-[(2-acetamido-5-propylthio)phenyl]-N"-(2-ethyl sulfonic acid)guanidine.

The compounds of the present invention exhibit useful anthelmintic activity while avoiding or reducing the disadvantages associated with known anthelmintics of the benzimidazole type, such as mebendazole, which are only sparingly water-soluble. Representative preferred compounds of its present invention have a water solubility of around 100 mg/ml or more compared with less than 1 mg/ml for mebendazole.

The compounds of the invention can be prepared by standard methods for the preparation of such guanidine-type compounds. According to a further feature of the invention, we provide a process for the preparation of a compound or salt as defined above, which comprises reacting a compound of the formula

$$H_2N(CH_2)_mR^2$$

with a compound of the formula

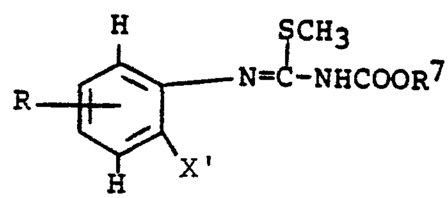

VI

whereafter the resulting compound of the formula

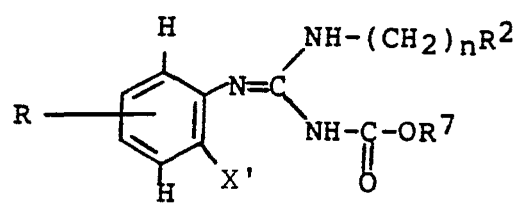

II

is if desired subjected to one or more of the following reaction steps in any appropriate order, where, in the above-defined reaction and the following steps, the various symbols R, $R^2$, $R^3$, $R^5$, $R^6$, $R^7$ and n are as defined above and X' is X defined above.

a) Hydrolytic removal of the group $NHCO_2R^7$ to yield a compound having an $-NH_2$ group in the guanidino group;

b) Acylation of a compound having an $-NH_2$ group in the guanidino group to a compound having instead an $-NHCONR^5R^6$ group;

c) Reaction of a compound having an $-NH_2$ group in the guanidino group with an inorganic isocyanate and then if desired with an alkylating agent, or with an alkyl isocyanate;

d) Oxidation of a compound having an $R^3S$-substituent in the phenyl group to a compound having a $R^3SO$- or $R^3SO_2$-substituent;

e) Reduction of a compound having a $2-NO_2$ group, if desired under alkylating conditions or with subsequent alkylation, to a compound having a 2-amino group;

f) Acylation of a compound having a 2-amino group;

g) Isolation of the final compound of formula I as such or as a pharmaceutically acceptable salt thereof.

A convenient intermediate for the preparation of compounds of formula I is an N-lower-alkoxycarbonyl-N'-[(2-X'-(4- or 5-)substituted)phenyl]-N"-(alkylacid)-guanidine, i.e. a compound of the formula

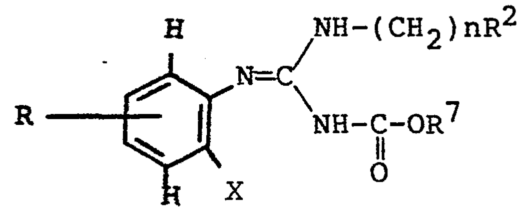

II

N-Alkoxycarbonyl compounds of formula II can be hydrolyzed according to well known procedures (e.g. with a base such as sodium hydroxide in a solvent such

as water or methanol) to prepare the corresponding guanidines (i.e. the $-CO_2R^7$ group is replaced by H).

Such guanidines can be treated with a diloweralkylcarbamyl chloride, e.g. dimethyl carbamyl chloride, according to techniques known in the art to obtain the corresponding N-[diloweralkylcarbamyl]-N'-[(2-nitro-(4- or 5-)substituted)phenyl]-N"-(substituted alkyl)-guanidine.

N-[Loweralkylcarbamyl]guanidines, e.g. methylcarbamyl-guanidines, can be prepared by treating guanidines having H instead of $-CO_2R^7$ with potassium cyanate followed by alkylation with a diloweralkyl sulfate, e.g. dimethylsulfate, or with a lower alkyl halide, e.g. methyl chloride or methyl bromide. Alternatively, N-[loweralkylcarbamyl]guanidines can be prepared by treating guanidines having H instead of $-CO_2R^7$ or the sodium salts thereof with an alkyl isocyanate.

If in the foregoing reaction alkylation is omitted and the product of the potassium cyanate reaction is hydrolysed with acid, e.g., hydrochloric, acetic or sulfuric acid, then the product will be an N-(carbamyl)-guanidine.

2-Nitro compounds of formula II can be used as intermediates in well known reactions to prepare compounds of the present invention, i.e. compounds wherein the substituent at the 2-position in the phenyl ring is of the formula $-NR^8R^9$.

For example, (2-lower alkylamino-(4- or 5-) substituted phenyl) compounds may be prepared by hydrogenation (catalytic or chemical) of compounds of formula II, followed by alkylation with a di-lower alkyl sulfate or a lower alkyl halide as described above.

Similarly, the lower alkylamino compounds prepared as in the immediately preceding paragraph can be further alkylated with a di-lower alkyl sulfate or a

lower alkyl halide to obtain the 2-di-loweralkylamino-(4- or 5-)substituted-phenyl compounds of formula I.

Alternatively, the conversion of the 2-nitro compound to the corresponding 2-alkylamino or 2-di-alkylamino compound can be carried out by hydrogenating the 2-nitro compound using an aliphatic aldehyde and platinum oxide as catalyst. For example, using formaldehyde in a 1:1 molar ratio to the nitro compound will yield the methylamine derivative, and using formaldehyde in a 2:1 molar ratio will yield the dimethylamine compound.

Compounds of formula I wherein one of $R^8$ and $R^9$ is lower alkyl and the other of $R^8$ and $R^9$ is lower alkoxycarbonyl can be prepared by the reaction of the appropriate 2-loweralkylamino-(4- or 5-)substituted-phenyl compound with the appropriately substituent acyl halide, e.g. methyl chloroformate, ethyl chloroformate, butyl chloroformate, 4-chlorophenyl chloroformate, 3-methylphenyl chloroformate, or 4-methoxyphenyl chloroformate.

Compounds of formula I wherein $R^8$ and $R^9$ are lower alkyl carbonyl or optionally substituted benzoyl can be prepared by the reaction of the appropriate carbonyl chloride (e.g. benzoyl chloride or acetyl chloride) with the desired 2-amino-(4 or 5-)substituted-phenyl compounds according to procedures known in the art.

Compounds of formula I wherein $R^8$ or $R^9$ is sulfolower alkyl or carboxy-lower alkyl can be prepared by the reaction of the corresponding Ω-halo-alkyl sulfonic or carboxylic acid salt with the intermediate formed by the hydrogenation of compounds of formula II. For example, the reaction of a hydrogenated compound of Cormula II with 2-chloroethane sulfonic acid sodium salt produces the taurine derivative (i.e. the N'-[(2-sulfonic acid ethylamino-(4- or 5-)substituted)-phenyl] compounds).

It will be apparent to those skilled in the art that successive modifications of compounds of formula II may be used to prepare compounds of formula I.

Compounds of formula II can be prepared by treating an appropriately substituted 2-X'-aniline with an alkoxycarbonyl-isothiocyanate, reacting the resulting product with a base such as sodium hydride or sodium methoxide and a dialkylsulfate or an alkylhalide to obtain the S-alkyl-isothiourea, and reacting this with an Ω-aminoalkylacid (e.g., taurine, 3-amino-1-propanesulfonic acid, 2-aminoethylphosphoric acid or 4-aminobutyric acid) and a base such as sodium hydroxide. A preferred embodiment of this reaction scheme, in which X' is $NO_2$, is shown below:

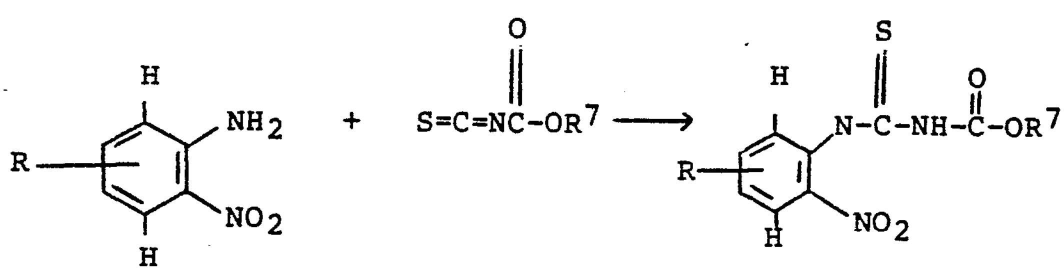

III IV V

V

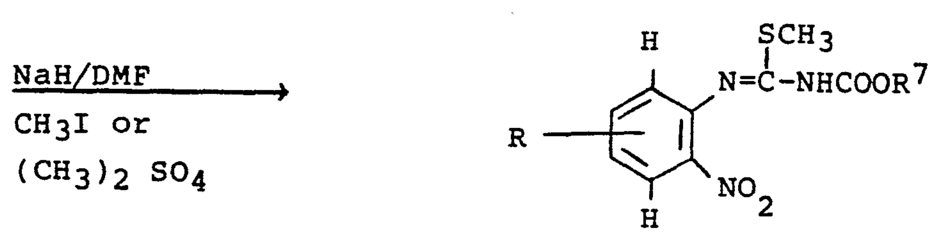


VI

IV $\xrightarrow[NaOH]{H_2N(CH_2)_nSO_3H}$ II

Compounds of formula III either are known in the art or may be prepared by methods well known to those skilled in the art.

Preferred compounds of formula I wherein R is $-S(O)_mR^3$ may be prepared by well known procedures from the corresponding $-SR^3$ compounds by treating the thio substituent with an oxidizing agent such as m-chloroperbenzoic acid, sodium m-periodate or hydrogen peroxide in acetic acid. Amino groups in the starting material may be protected during the oxidation by the addition of a reagent such as trifluoroacetic acid. Treatment with one equivalent of oxidizing agent will yield the $-SOR^3$ (sulfinyl) compound while treatment with two equivalents will yield the $-SO_2R^3$ compound (sulfonyl). This oxidation may be carried out at one of several stages in the preparation of the compounds as shown below, wherein the preparation of a sulfinyl compound is described:

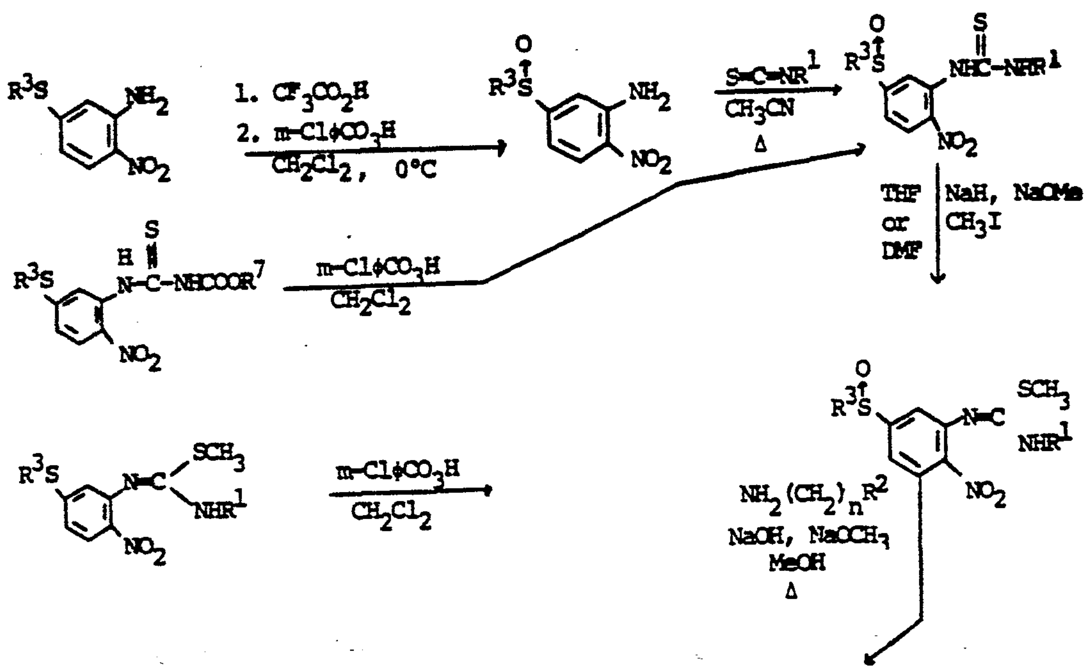

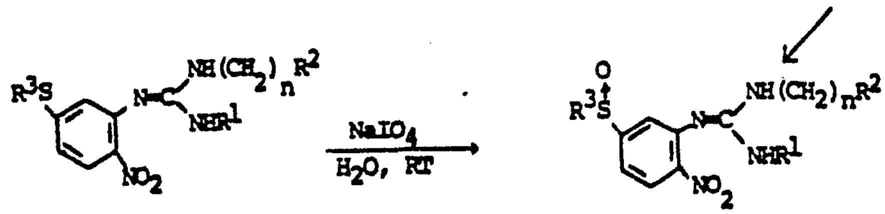


The nitro group can, of course, be replaced with any appropriate group comprised within X', especially and N-diloweralkyl group.

2-Amino-compounds of formula II can be prepared as follows: For example, 3,4-dinitrochlorobenzene may be treated with liquid ammonia in a solvent such as ethanol to obtain 2-nitro-5-chloro-aniline, which may then be treated with acetic anhydride in a solvent such as pyridine at 0-5°C to yield 2-nitro-5-chloroacetanilide. The 2-nitro-5-chloro acetanilide may then be converted to the corresponding 2-dialkylamino-5-chloroacetanilide by hydrogenating the nitro compound using platinum on carbon as catalyst in the presence of an aliphatic aldehyde (2:1 molar ratio to the nitro compound). The acetanilide can be hydrolyzed with a reagent such as sodium methoxide in a solvent such as methanol to give the corresponding 2-dialkylamino-5-chloroaniline. The chloroaniline can be treated with an appropriate reagent to give a 2-dialkylamino-5-substituted-aniline, e.g. the chloroaniline reacted with 1-propanethiol and sodium hydroxide in dimethylformamide/water at room temperature will yield 2-dialkylamino-5-propylthioaniline. This aniline may then be treated as the compound of formula III in the foregoing reaction scheme to obtain a 2-dialkylamino-compound analogous to the compound of formula II.

Metal salts, alkyl ammonium salts and other pharmaceutically acceptable salts may be prepared according to methods well known to those skilled in the art.

The following examples illustrate the preparation of compounds and compositions of this invention.

EXAMPLE 1

N-[(2-Nitro-5-propylthio)phenyl]-N'-(2-ethyl sulfonic acid)guanidine

Combine N-methoxycarbonyl-N'-[(2-nitro-5-propylthio)phenyl]-N"-(2-ethyl sulfonic acid)guanidine (42.1g) and sodium hydroxide (17.6 g of a 50% solution) in water (420 ml) and reflux for 4 hours. Cool the resulting solution and acidify to pH 1-2 with 10N aqueous hydrochloric acid. Filter off the resulting precipitate, wash with cold water (3 x 100 ml) then methanol (2 x 100 ml) and dry to obtain the title compound, m.p. 250-253°C (dec).

EXAMPLE 2

N-(N"'-Dimethylcarbamyl)-N'-[(2-nitro-5-propylthio)-phenyl]-N"-(2-ethyl sulfonic acid)guanidine

Method A: Suspend the product of Example 1 (3.62 g) in anhydrous dimethylformamide (100 ml), add sodium methoxide (0.54 g) and warm gently with stirring to make a homogenous solution. Add N,N-dimethyl carbamoyl chloride and heat at 80°C for 8 hours. Cool the resultant mixture at room temperature, pour into water (100 ml) and stir 1 hour. Filter off the resulting solid, wash the filtrate with cold acetone and dry the filtrate to obtain the title compound.

Method B: Alternatively, the title compound may be prepared as follows:

Combine potassium thiocyanate (10.2 g) and dimethyl carbamoyl chloride (10.7 g) in anhydrous acetonitrile (250 ml) and reflux for 8 hours. Filter the resulting mixture and combine the filtrate with 2-nitro-5-propylthioaniline (14.2 g) and reflux for 8 hours. Evaporate the solvent and dissolve the resultant residue in anhydrous dimethylformamide (100 ml). Cool the solution to 0-5°C and add sodium hydride (2.0 g) followed by iodomethane (30 g), then reflux again for 8 hours. Cool the resultant mixture to room temperature, filter and add the sodium salt of taurine, stir for 4 hours, then filter off the resulting precipitate to obtain the title compound.

EXAMPLE 3

N-Methoxycarbonyl-N'-[(2-dimethylamino-5-propylthio)-phenyl]-N"-(2-ethyl sulfonic acid)guanidine

Combine N-methoxycarbonyl-N'-[(2-nitro-5-propylthio)phenyl]-N"-(2-ethyl sulfonic acid)guanidine (4.2g) Raney Nickel (1.0-2.0 g) and formaldehyde (0.6 g) in 95% ethanol (100 ml) in a pressure bottle and catalytically reduce the mixture at an initial pressure of 3 atm. hydrogen. After 0.03M to 0.04M hydrogen is absorbed, stop the reduction and filter off the catalyst. Evaporate the filtrate to dryness to obtain the title compound.

EXAMPLE 4

N-[(2-Dimethylamino-5-propylthio)phenyl]-N'-(2-ethyl sulfonic acid)guanidine

Combine the product of Example 3 with sodium hydroxide in water and reflux for 4 hours. Cool the resulting solution and acidify to pH 1-2 with 10N

aqueous hydrochloric acid. Filter the resulting precipitate, wash with cold water (3 x) then methanol (2 x) and dry to obtain the title compound.

EXAMPLE 5

N-Methoxycarbonyl-N'-[(2-dimethylamino-5-propylsulfinyl)-phenyl]-N"-(2-ethane sulfonic acid)guanidine

A. In the pressure bottle of an apparatus for catalytic reduction, combine N-methoxycarbonyl-N'[(2-nitro-5-propyl-thio)phenyl]-S-methyl-isothiourea (3.4g), formaldehyde (0.6 g), 95% ethanol (100 ml) and Raney nickel (1.0 to 2.0 g), evacuate the bottle and apply an initial pressure of 3 atm. of hydrogen. After 0.03 to 0.04 M hydrogen is absorbed (1-2 hours), stop the reduction and filter off the catalyst. Evaporate the resultant filtrate to dryness and recrystallize the resultant residue to obtain N-methoxycarbonyl-N'-[(2-dimethylamino-5-propylthio)phenyl]-S-methyl-isothiourea.

B. Slurry the product of step A (1.7 g) in ether (50 ml), add *m*-chloroperbenzoic acid (1.1 g of an 85% solution in 20 ml ether) and stir at room temperature 20 minutes. Extract the resultant solution with 5% aqueous sodium bicarbonate solution (3 x 50 ml), dry the organic phase over magnesium sulfate and evaporate the solvent. Purify the resultant crude product by chromatography and recrystallize the chromatographed product from ether to obtain N-methoxycarbonyl-N'-[(2-dimethylamino-5-propylsulfinyl)phenyl]-S-methyl isothiourea.

C. To a solution of sodium taurine (1.75 g) in water (10 ml) at room temperature, add a solution of the product of step B (3.3 g) in methanol (100 ml) and stir for several days. Filter off the solid and wash it with acetone. Add the solid to boiling methanol, filter off

the excess insoluble sodium taurine and refrigerate the methanol solution. Filter off the resultant precipitate and dry it to obtain N-methoxycarbonyl-N'-[(2-dimethylamino-5-propyl-sulfinyl)-phenyl]-N"-(2-ethyl sulfonic acid)guanidine sodium salt. To obtain the free Zwitterion, dissolve the sodium salt in water, adjust to pH 1.5 with acid (e.g. hydrochloric) and filter off the resultant solid. Wash the resultant solid with acetone and dry to obtain the title compound.

## EXAMPLE 6

### N-[(2-Nitro-5-propylsulfonyl)phenyl]-N'-(2-ethyl sulfonic acid)guanidine

In a 500 ml 3-neck round bottom flask, equipped with a magnetic stirrer, thermometer and dropping funnel, there was placed 9.05 g (0.025M) of N-[(2-nitro-5-propylthio)phenyl]-N'-(2-ethyl sulfonic acid)guanidine and 150 ml of glacial acetic acid. The suspension was cooled to about 10°C and 6.5 ml of 30% aqueous hydrogen peroxide solution was added slowly. A slightly ethoxthermic reaction occurred. The mixture was allowed to warm to room temperature and stirred at room temperature for about 48 hours. The reaction mixture was monitored periodically; towards the completion of the reaction it was almost a clear solution. Then particulate matter was filtered off and the filtrate was concentrated to dryness under vacuum. The residue was dissolved in 2 ml of water and cooled, and 50 ml of acetone was added. The resulting light yellow crystalline solid was filtered off and washed with acetone and dried; m.p. 240-244°C (dec.); yield about 9.0 g (91.8%).

The identity of the compound was confirmed by HPLC and by its NMR, IR and mass spectra.

EXAMPLE 7

N-[(2-Nitro-5-propylsulfinyl)phenyl]-N'-(2-ethyl sulfonic acid)guanidine

In a 500 ml 3-neck round bottom flask, equipped with a stirrer, thermometer and dropping funnel, there was placed 9.05 g (0.025M) of N-[(2-nitro-5-propylthio)phenyl]-N'-(2-ethyl sulfonic acid)guanidine and 150 ml of glacial acetic acid. The suspension was cooled to about 10°C and 3.3 ml of 30% aqueous hydrogen peroxide solution was very slowly added dropwise over a period of several hours. Meanwhile, the mixture was allowed to warm to room temperature. After the addition was complete, the mixture was stirred at room temperature for about 60 hours or until high pressure liquid chromatography showed that no more starting material was present. Towards the end of the reaction the mixture became almost clear. Particulate matter was then filtered off and the filtrate was concentrated to dryness under vacuum. The residue was dissolved in about 2 ml of water and about 50 ml of acetone was added. The resulting light yellow crystalline solid was filtered off and washed with acetone and dried; m.p. 242-250°C; yield about 8.5 g. The nature of the compound was confirmed by HPLC, NMR, IR and its mass spectrum and by its analysis:

Found: C 37.93%, H 4.68%, N 14.68%, S 16.98%;

$C_{12}H_{18}N_4O_5S_2$ requires C 38.09%, H 4.97%, N 14.81% and S 16.94%.

Using the methods described above, the following compounds also can be prepared:

N-methoxycarbonyl-N'-[(2-methylamino-(4 or 5)-propylthio)-phenyl]-N"-(2-ethyl sulfonic acid)guanidine;

N-methoxycarbonyl-N'-[(2-dimethylamino-(4 or 5)-propylthio)-phenyl]-N"-(2-ethyl sulfonic acid)guanidine;

N-methoxycarbonyl-N'-[(2-sulfonic acid ethyl)amino-(4 or 5)-propylthio)-phenyl]-N"-(2-ethyl sulfonic acid) guanidine;

N-(4-chlorobenzyloxycarbonyl)-N'-[(2-carboxyethylamino-(4 or 5)-propylthio)phenyl]-N"-(2-ethyl sulfonic acid)guanidine;

N-[(N'''methyl-N'''-benzyl)carbamyl]-N'-[(2-amino-(4 or 5)-propylthio)phenyl]-N"-(2-ethyl sulfonic acid)-guanidine;

N-[(N'''methyl-N'''-ethyl)carbamyl]-N'-[(2-amino-(4 or 5)-propoxy)phenyl]-N"-(2-ethyl sulfonic acid)guanidine;

N-(4-chlorobenzyloxycarbonyl)-N'-[2-amino-(4 or 5)-chlorophenyl]-N"-(2-ethyl phosphoric acid)guanidine;

N-dimethylcarbamyl-N'-[(2-amino-(4 or 5)propoxy)phenyl]-N"-(2-ethyl hydrogen phosphate)guanidine;

N-methoxycarbonyl-N'-[(2-amino-(4 or 5)-(3-hydroxy)propyloxy)phenyl]-N"-(2-ethyl sulfonic acid)guanidine;

N-carbamyl-N'-[(2-amino-(4 or 5)-amidophenyl]-N"-(2-ethylsulfonic acid)guanidine;

N-methoxyethylcarbamyl-N'-[(2-amino-(4 or 5)-(4-acetylphenyl)]-N"-(2-ethyl sulfonic acid)guanidine;

N-methoxycarbonyl-N'-[(2-amino-(4 or 5)-propylsulfonyl)-phenyl]-N"-(2-ethyl sulfonic acid)guanidine;

N-ethoxycarbonyl-N'-[(2-methylamino-(4 or 5)-cyclopropyl-thio)phenyl]-N"-(2-ethyl sulfonic acid)guanidine;

N-carbamyl-N'-[(2-dimethylamino-(4 or 5)-(3-ethoxy)-propylthio)phenyl]-N"-(2-ethyl hydrogen sulfate)guanidine;

N-carbamyl-N'-[(2-amino-(4 or 5)-phenylthio)phenyl]-N"-(2-ethyl sulfonic acid)guanidine;

N-(2-hydroxyethylcarbamyl)-N'-[(2-acetamido-(4 or 5)-propylthio)phenyl]-N"-(2-ethyl sulfonic acid)guanidine;

N-methoxyethylcarbamyl-N'-[(2-methoxycarbonylamino-(4 or 5)-4-chlorophenylthio)phenyl]-N"-(2-ethyl sulfonic acid)guanidine;

N-phenoxycarbonyl-N'-[(2-(2-methoxyethyl)amino-(4 or 5)-methoxybenzylthio)phenyl]-N"-(2-ethyl sulfonic acid)guanidine;

N-methoxycarbonyl-N'-[(2-anilino-(4 or 5)-butylsulfinyl)phenyl]-N"-(2-ethyl sulfonic acid)guanidine;

N-[(2-benzamido-(4 or 5)-propylthio)phenyl]-N'-(2-ethyl sulfonic acid)guanidine;

N-methylcarbamyl-N'-[(2-(4-chloroanilino)-(4 or 5)-propylthio)phenyl]-N"-(2-ethyl sulfonic acid)guanidine;

N-dimethylcarbamyl-N'-[(2-(4-methoxybenzamido)-(4 or 5)-propylthio)phenyl]-N"-(2-ethyl carboxylic acid)guanidine;

N-carbamyl-N'-[(2-nitro-(4 or 5)-(3-ethoxy)propylthio)-phenyl]-N"-(2-ethyl sulfonic acid)guanidine;

N-methoxycarbonyl-N'-[(2-nitro-(4 or 5)-(3-hydroxy)propylthio)-phenyl]-N"-(2-ethyl carboxylic acid)guanidine;

N-carbamyl-N'-[(2-nitro-(4 or 5)-phenylthio)phenyl]-N"-(2-ethyl sulfonic acid)guanidine;

N-(2-hydroxyethylcarbamyl)-N'-[(2-nitro-(4 or 5)-4-chlorophenylthio)phenyl]-N"-(2-ethyl sulfonic acid)guanidine;

N-methoxyethylcarbamyl-N'-[(2-nitro-(4 or 5)-(2,3 or 4-methoxybenzyl)thio)-phenyl]-N"-(2-ethyl sulfonic acid)guanidine;

N-methoxycarbonyl-N'-[(2-nitro-(4 or 5)-2-pyridylthio)-phenyl]-N"-(2-ethyl carboxylic acid)guanidine;

N-phenoxycarbonyl-N'-[(2-nitro-(4 or 5)-(4-trifluoromethylphenyl)thio)phenyl]-N"-(2-ethyl sulfonic acid)guanidine;

N-[(N'''-methyl-N'''-benzyl)carbamyl]-N'-[(2-nitro-(4 or 5)-butylsulfinyl)-phenyl]-N"-(2-ethyl hydrogen sulfate)guanidine;

N-methoxycarbonyl-N'-[(2-nitro-(4 or 5)-trifluoromethylphenyl]-N"-(2-ethyl sulfonic acid)guanidine;

N-carbamyl-N'-[(2-nitrophenyl)-N"-(2-ethyl sulfonic acid)guanidine;

N-(4-chlorobenzyloxycarbonyl)-N'-(2-nitro-(4 or 5)-chlorophenyl)-N"-(2-ethyl phosphoric acid)guanidine;

N-dimethylcarbamyl-N'-[(2-nitro-(4 or 5)-propoxy)-phenyl]-N"-(2-ethyl sulfonic acid)guanidine;

N-[N'''-methyl-N'''-ethyl)carbamyl]-N'-[(2-nitro-(4 or 5)-(3-hydroxypropyloxy))phenyl]-N"-(2-ethyl sulfonic acid)guanidine;

N-carbamyl-N'-[(2-nitro-(4 or 5)-amidophenyl)-N"-(2-ethyl sulfonic acid)guanidine; and

N-methoxyethylcarbamyl-N'-[(2-nitro-(4 or 5)-4-acetylphenyl)-N"-(2-ethyl sulfonic acid)guanidine.

The compounds of the present invention are useful in combatting helminthiasis, i.e. in treating animals, including humans, suffering from an infestation of parasitic worms, for example, roundworms, hookworms, whipworms or tapeworms, by administering to the host animal a therapeutic amount of a compound of the present invention.

The compounds of this invention exhibit useful anthelmintic effects when administered to a host (e.g. a human, swine, dog, bird or ruminant) at doses as low as about one milligram per kilogram of body weight to about one hundred fifty milligrams per kilogram in a single day dosing or over several days, according to techniques well known in the art. A preferred method is to administer the compound at 5 to 25 milligrams per

kilogram in a single dose. Regarding toxicity, the compounds of this invention are non-toxic at the therapeutic dose.

The optimum dose for each species of animal and for each type of parasite can readily be determined by one skilled in the art of using standard techniques such as the Modified McMaster Egg Counting Technique as described by H.B. Whitlock and H. McL. Gordon, J. Council Scientific Industrial Research (Australia) 12, p. 50, 1939 and H.B. Whitlock, J. Council Scientific Research (Australia) 21, p. 177, 1948.

From these, and similar tests, anthelmintic efficacy is assessed by determining the number of eggs in feces passed on the days following treatment with the compound compared with pretreatment days. Based on experimentation, proper dosages for curing various infections can be determined.

Compounds of this invention may be administered in various formulations well known to those skilled in the human and veterinary medical arts, e.g., solutions, capsules, tablets and injectable preparations. In addition, for veterinary use, the compounds may be administered as feed or drinking water additive preparations.

For injectable preparations, the active ingredient is admixed with suitable sterile carriers such as sterile water and isotonic saline solution.

Suitable clinical formulations containing the compounds of this invention can be administered orally in the form of tablets, capsules, elixirs and the like. The active compound is compounded with inert carriers such as, for example, gums, starches and sugars or it may be incorporated into gelatin capsules or formulated into elixirs which have the advantage of being amenable to manipulations in flavor by the addition of flavoring agents.

Anthelmintic formulations particularly useful for, but not limited to, veterinary use comprise the compounds of this invention in ready to use liquid suspensions or wettable or water-dispersible powders which are dissolved in water prior to use.

The following examples show particularly useful formulations. In the examples, the term "Drug" refers to a compound in accordance with this invention.

A. Liquid-suspension formulation:

A liquid-suspension formulation may contain from 50 to 55% w./v. (grams/liters) of the active compound together with a dispersing agent and stabilizing agent. A typical formulation is as follows:

Drug..........................50 to 55 parts by weight
Dispersing agent .............. 1/2 to 2 parts by weight
Stabilizing agent ................ 1 to 3 parts by weight
Preservative ....................... as required
Water .............................. Sufficient to make 100 volumes.

Suitable dispersing agents are those containing sulphonate groups, for example sodium lignin sulphonate, or the sulphonated phenol or naphthol formaldehyde polymers. Bentonite may be employed as the stabilizing agent, although it is possible to use such protective colloids as carboxymethyl cellulose, sodium alginate and the like. The formulations can be prepared by mixing the active compound and water containing dissolved dispersing agents very vigorously by means of suitable mechanical mixing equipment.

B. Powder formulation:

A wettable or water-dispersible powder formulation may contain about 90 to 95% w./w. of the active compound together with a wetting agent and dispersing agent. A diluent such as kaolin can also be added if a concentration below about 98% w./w. is required. An anti-foaming agent and, in some cases, a stabilizing agent may be present. A typical formulation is as follows:

Drug .......................... 90 to 95 parts by weight
Wetting agent .................. 1/2 to 4 parts by weight
Stabilizing agent ................ 0 to 2 parts by weight
Anti-foaming agent .................. 0.01 to 1 by weight
Water ............................... 0 to 5 by weight

Suitable wetting agents are the non-ionic alkylphenol-ethylene oxide adducts, such as an octylphenol or nonylphenol condensed with ten moles of ethylene oxide, or anionic materials, such as the synthetic aryl alkyl sulphonates, or sodium dibutyl napthalene sulphonate. In general, about 1% w./w. wetting agent is required. The anti-foaming agent employed may be either a silicone or such materials as ethyl hexanol, octanol and the like; and the stabilizing agent may be chosen from bentonite or the water-soluble gums as discussed above. Wettable or water-dispersible powder formulations are prepared by careful and adequate mixing of the active compound with other ingredients with or without the addition of some water using typical powder blending equipment such as a ribbon blender. The powder is dissolved in water by the user before application in the field.

| C. Tablet formulation | Grams per 1000 tablets |
| --- | --- |
| Drug | 200.0 |
| Lactose | 90.0 |
| Dicalcium phosphate, hydrous | 122.5 |
| Polyvinylpyrolidone | 25.0 |
| Polyethyleneglycol 1500 | 7.5 |
| Corn Starch | 50.0 |
| Magnesium Stearate | 5.0 |
| | 500.0 |

Mix the active compound, the lactose and the dicalcium phosphate. Dissolve the polyethyleneglycol 1500 and the polyvinylpyrrolidone in approximately 20 ml of water. Granulate the powder blend with the water solution, adding additional water if necessary, to product a damp mass. Pass the wet granulation through a 12 mesh screen; spread on trays and air dry at 35° C. Blend the dry granulates with the starch and the magnesium stearate. Compress into 500 mg tablets.

| D. Capsule formulation | Grams per 1000 capsules |
| --- | --- |
| Drug | 200.0 |
| Lactose | 198.0 |
| Magnesium Stearate | 2.0 |
| | 400.0 |

Blend the ingredients and fill into hard gelatine capsules.

| E. Injectable formulation | mg/ml |
| --- | --- |
| Drug | 50.0 |
| Polyethylene Glycol 400 | 500.0 |
| Dimethyl Acetamide | 300.0 |
| Benzyl Alcohol | 20.0 |
| Water for Injection to q.s. | 1.0 ml |

Combine the above ingredients using standard techniques.

CLAIMS

1. Compounds of the formula

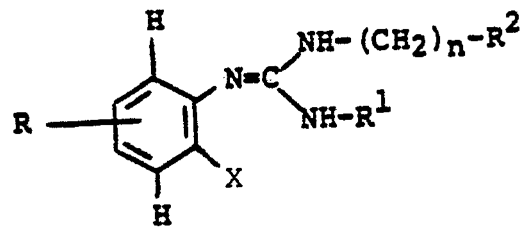

I.

including their tautomers at the guanidine group,

and the pharmaceutically acceptable salts thereof,

wherein X is a group $NO_2$ or $NR^8R^9$,

R is hydrogen, $-OR^3$, $-S(O)mR^3$, $-\overset{O}{\overset{\|}{C}}-\overset{R^3}{\overset{|}{N}}-R^4$, $-\overset{O}{\overset{\|}{C}}-R^3$, halogen or $-CF_3$;

R1 is hydrogen, $-\overset{O}{\overset{\|}{C}}-\overset{R^5}{\overset{|}{N}}-R^6$, or $-\overset{O}{\overset{|}{C}}-OR^7$;

$R^2$ is $-SO_3H$, $-OSO_3H$, $-COOH$, $-PO_3H_2$, or $-OPO_3H_2$;

$R^3$ and $R^4$ are independently hydrogen, loweralkyl, loweralkoxyloweralkyl, hydroxyloweralkyl, cycloloweralkyl, phenyl or substituted phenyl, benzyl or substituted benzyl (wherein there are 1, 2 or 3 substituents on the substituted phenyl or substituted benzyl selected independently from halogen, loweralkyl, loweralkoxy, haloloweralkyl, or loweralkoxyloweralkyl), or 5 or 6 membered heterocycles having 1 or 2 heteroatoms selected independently from oxygen, nitrogen and sulfur;

$R^5$, $R^6$ and $R^7$ are independently hydrogen, loweralkyl, loweralkoxyloweralkyl, hydroxyloweralkyl, phenyl or substituted phenyl, and benzyl or substituted

benzyl (where there are 1, 2 or 3 substituents on the substituted phenyl or substituted benzyl selected independently from halogen, loweralkyl, loweralkoxy, haloloweralkyl, or loweralkoxyloweralkyl) except that $R^7$ cannot be hydrogen;

$R^8$ and $R^9$ are independently hydrogen, loweralkyl, loweralkylcarbonyl, loweralkoxyloweralkyl, loweralkoxyloweralkyl, phenyl or substituted phenyl, and benzyl or substituted benzyl phenoxycarbonyl or substituted phenoxycarbonyl (wherein there are 1, 2 or 3 substituents on the substituted phenyl, substituted benzoyl or substituted phenoxycarbonyl independently selected from halogen, lower alkyl, loweralkoxy, trifluoromethyl, and haloloweralkyl), carboxy-substituted lower alkyl, or sulfo-substituted lower alkyl;

$\underline{n}$ is 1 to 6; and

$\underline{m}$ is 0, 1 or 2;

provided that, when R is loweralkoxy, loweralkylthio, loweralkylsulfoxy, loweralkylsulfonyl, phenoxy, phenylsulfonyl, phenylthio or phenylsulfoxy;

$R^1$ is loweralkoxycarbonyl, loweralkoxylower-alkoxycarbonyl or hydroxyloweralkoxycarbonyl, and

X is nitro, loweralkylcarbonylamino, phenylacetamido, benzamido, or benzamido substituted with loweralkyl or halogen,

then $R^2$ is $PO_3H_2$ or $OPO_3H_2$.

2. A compound as claimed in claim 1 wherein R is at the 5-position, especially a 5-$S(O)_mR^3$ or 5-$CF_3$ group.

3. A compound as claimed in claim 1 wherein R is a 5-$SR^3$ group, in particular where $R^3$ is a lower alkyl, especially a propyl, group.

4. A compound as claimed in any of claims 1 to 3 wherein $R^2$ is $-SO_3H$ and n is 2 or 3.

5. A compound as claimed in any of claims 1 to 4 wherein X is $NO_2$ or $NH_2$.

6. A compound as claimed in any of claims 1 to 5 wherein $R^1$ is hydrogen or a group $CONR^5R^6$, wherein $R^5$ and $R^6$ are lower alkyl, e.g., methyl groups.

7. N-[(2-Nitro-5-propylsulfonyl)phenyl]-N'-(2-ethyl sulfonic acid)guanidine,

N-[(2-nitro-5-propylthio)phenyl]-N'-(2-ethyl sulfonic acid)guanidine, and

N-[(2-nitro-5-propylsulfinyl)phenyl]-N'-(2-ethyl sulfonic acid)guanidine.

8. N-Methoxycarbonyl-N'-[(2-dimethylamino-5-propylthio)phenyl]-N"-(2-ethyl sulfonic acid)guanidine,

N-methoxycarbonyl-N'-[2-dimethylamino)-5-propylthio)phenyl]-N"-(3-propyl sulfonic acid)guanidine,

N-methoxycarbonyl-N'-[(2-(N"'-methyl-N"'-(2-ethyl sulfonic acid)amino)-5-propylthio)phenyl]-N"-(2-ethyl sulfonic acid)guanidine, and

N-methylcarbonyl-N'-[(2-acetamido-5-propylthio)phenyl]-N"-(2-ethyl sulfonic acid)guanidine.

9. A process for the preparation of a compound or salt claimed in claim 1,
which comprises reacting a compound of the formula

$$H_2N(CH_2)_nR^2$$

with a compound of the formula

VI

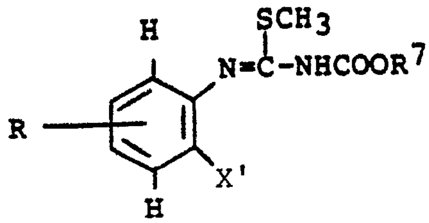

whereafter the resulting compound of the formula

II

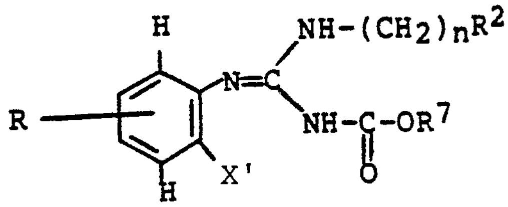

is if desired subjected to one or more of the following reaction steps in any appropriate order, wherein, in the above-defined reaction and the following steps, the various symbols R, $R^2$, $R^3$, $R^5$, $R^6$, $R^7$ and n are as defined in claim 1, and X' is X as defined in claim 1:

a) Hydrolytic removal of the group $NHCO_2R^7$ to yield a compound having an $-NH_2$ group in the guanidino group;

b) Acylation of a compound having an $-NH_2$ group in the guanidino group to a compound having instead an $-NHCONR^5R^6$ group;

c) Reaction of a compound having an $-NH_2$ group in the guanidino group with an isocyanate isocyanate and then if desired with an alkylating agent, or with an alkyl is isocyanate;

d) Oxidation of a compound having an $R^3S$-substituent in the phenyl group to a compound having an $R^3SO$- or $R^3SO_2$-substituent;

e) Reduction of a compound having a $2-NO_2$ group, if desired under alkylating conditions or with subsequent alkylation, to a compound having a 2-amino group;

f) Acylation of a compound having a 2-amino group;

g) Isolation of the final compound of formula I as such or as a pharmaceutically acceptable salt thereof.

10. A composition comprising an anthelmintically effective amount of a compound of claim 1 and a pharmaceutically acceptable carrier.

European Patent Office

**EUROPEAN SEARCH REPORT**

0147537

Application number

EP 84 11 1272

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| A | EP-A-0 050 286 (SCHERING CORP.) *The whole document* ----- | 1-10 | C 07 C 149/437<br>C 07 C 147/14<br>C 07 C 147/12<br>A 61 K 31/185<br>A 61 K 31/27 |
| | | | TECHNICAL FIELDS SEARCHED (Int. Cl.4) |
| | | | C 07 C 149/00<br>C 07 C 147/00 |

The present search report has been drawn up for all claims

| Place of search | Date of completion of the search | Examiner |
|---|---|---|
| THE HAGUE | 18-12-1984 | GAUTIER R.H.A. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document